# EUROPEAN PATENT SPECIFICATION

(11) **EP 1 299 041 B1**
(45) Date of publication and mention of the grant of the patent: **04.10.2006**
(21) Application number: 00961808.3
(22) Date of filing: 12.09.2000
(51) Int. Cl.: A61B 17/70, A61B 17/68

(54) **INTERVERTEBRAL DISC REPAIR**
REPARATUR VON ZWISCHENWIRBELSCHEIBEN
REPARATION D'UN DISQUE INTERVERTEBRAL

(30) Priority: 12.06.2000 US 211125 P
(43) Date of publication of application: 09.04.2003
(73) Proprietor: Yeung, Jeffrey E., San Jose, CA 95127 (US)
(72) Inventor: Yeung, Jeffrey E., San Jose, CA 95127 (US)
(74) Representative: Stainthorpe, Vanessa Juliet
(86) International application number: PCT/US2000/024921
(87) International publication number: WO 2001/095818

(56) References cited:
- WO-A-95/31946
- WO-A-99/37219
- US-A- 2 485 531
- US-A- 5 893 850

## Description

This invention relates to a toggle compressive device for treating bulging discs, intervertebral instability or spinal stenosis.

### BACKGROUND, TRADITIONAL SURGICAL PRACTICES AND PRIOR INVENTIONS

### Low Back Pain

Low-back pain is one of the most prevalent, costly and debilitating ailments afflicting mankind. Seventy to eighty-five percent of all people have back pain at some time in their life. Symptoms are most common among middle-aged adults and are equally common in both men and women. Back pain related to disc disorders, however, is more prevalent among men. The recurrence rate of low back pain ranges from 20% to 44% annually, with lifetime recurrences of 85% (National Institute of Health Guide, Vol. 26, 16 May 1997).

Low back pain is very costly to patients, our health care system and society. For many, no position can ease their pain or numbness, not even bed rest. It is often the reason for decreased productivity due to loss of work hours, addiction to pain-killing drugs, emotional distress, prolonged hospital stays, loss of independent living, unplanned early retirements and even financial ruin. Each year in the US, about 2% of the work force have back injuries covered by worker's compensation, with about $12 billion spent directly on medical costs in 1994.

Most back pain is caused by a defect or damage in the intervertebral disc. The disc is comprised of nucleus pulposus and annulus. The nucleus pulposus is highly gelatinous with a composition of 70-90% water, 25-60% proteoglycan (dry weight) and 10-20% collagen (dry weight). The function of the nucleus pulposus is to sustain prolonged compression during the day and to resiliently re-inflate and reestablish disc height during the night. The pulposus is retained and surrounded by layers of cartilaginous annulus. Together the pulposus and the annulus behave as a resilient cushion. In the erect position, the weight of the body constantly compresses upon a stack of these cushions alternating between a series of vertebrae. During constant compression,
the pulposus in each disc also behaves as a water reservoir, which is slowly and constantly being squeezed and drained of its water content through the end plates connected to the vertebrae. As a result, the disc height decreases throughout the day. During bed rest, the weight of the body no longer compresses the disc. Due to the water absorbing nature of the nucleus pulposus, the flow of water is now reversed from the vascular vertebrae back into the proteoglycan and collagen. As a result, the disc height is reestablished, ready to provide support for another day.

Under dynamic conditions, the gelatinous nucleus pulposus exhibits predominantly solid-like behavior with values for dynamic modulus ranging from 7 to 20 kPa (J.C. Iatridis et. al., J. Biomechanics, Vol. 30, No. 10, 1005-1013, 1997). With aging and degeneration, the viscoelastic property of the nucleus pulposus undergoes a transition from fluid-like to solid-like behavior (J.C. Iatridis et. al., Journal of Orthopaedic Research, 15:318-322, 1997). As a result, both the resiliency and disc height diminish.

Some causes that contribute to low back pain are classified. Type I: Acute back sprain involves damage to ligaments, muscles or even the vertebral end plates from physical overload. Type II: Organic idiopathic spine pain occurs from increased fluid uptake by the disc. Type III: Posteriolateral annulus disruption of annular fibers irritates nerves associated with the sacroiliac region, buttock and the back of the thigh. This situation may resolve itself through reabsorption or neutralization by phagocytosis of the disrupted annular fibers. Type IV: Nerve root irritation by the bulging disc leads to sciatica. This type of disc protrusion is traditionally repaired surgically by tissue removal, chemonucleolysis or percutaneous discectomy. Type V: Nerve irritation by wandering sequestered disc material has unpredictable exacerbation and remissions. Type VI: Sequestrum of the annulus and/or nucleus into the spinal canal or intervertebral foramen results in nerve irritation from inflammation, mechanical pressure, chemical irritation, autoimmune response or combinations of irritants. Type VII - A degenerated disc, with substantial decrease in mechanical properties, is often associated with pain and disability.

The most common reason for recurrent pain is the bulging or herniation of an intervertebral disc. The traditional surgical treatment for a bulging or herniated disc is a series of tissue removing, filling and supporting procedures: (1) laminectomy, removal of lamina from the vertebra which covers part of the herniated disc, (2) discectomy, removal of the disc, (3) bone harvesting usually from the patient's iliac crest, (4) bone cement filling of the donor site, (5) donor bone packing into the vacant disc space, (6) supporting adjacent vertebral bodies with rods, connectors, wire and screws, and finally (7) closing multiple surgical sites.

After a discectomy, numerous postoperative complications can occur. The major ones are lumbar scarring and vertebral instability. The scar tissue extends and encroaches upon the laminectomy site and intervertebral foramen, then once again, pain returns, which leads to more surgery. In fact, repeat operations are very common. Unfortunately, the success rates of repeat operations are often less, in some cases, far less than the first. More operations lead to more scarring and more pain. Current recommendations to the patients are to avoid surgical procedures unless the pain and inconveniences are absolutely unbearable.

Even for the fortunate patients with long term success following discectomies performed twenty years ago, their isokinetic test results clearly indicate weaknesses compared to populations without discectomies.

There was and still is increasing interest in more effective and less invasive surgical techniques on the spine to reduce both trauma and cost. The major objectives of surgery on bulging or herniated lumbar discs are (1) decompression of the involved nerve root or roots, and (2) preservation of bony spine, joints and ligaments.

Chymopapain is an enzyme used to digest away the nucleus pulposus, the viscous and gel-like substance in the central portion of the disc, which then creates space for the bulging part of the disc to pull back from the encroached nerve root. The needle for injecting the chymopapain is accurately guided to the mid-portion of the disc by a stereotaxic device. The overall success rate is documented as high as 76%. However, some patients are allergic to the treatment and die from anaphylaxis. Some others suffer from serious neuralgic complications, including paraplegia, paresis, cerebral hemorrhage and transverse myelitis.

Percutaneous nuclectomy is an alternative method for removing nucleus pulposus without the allergic reaction of chymopapain; and it rarely causes epidural scarring. Similar to chymopapain injection, a needle followed by a tube-like instrument is guided and confirmed by anteroposterior and lateral fluoroscopy. The nucleus pulposus is then removed mechanically or by vacuum. As a result, a void is created within the disc and the bulging decreases, like the air being released from a worn out tire, with the hope that the bulging portion of the disc will recede and no longer encroach upon the adjacent nerve root. This type of procedure is often referred to as a decompression procedure. However, the success of the percutaneous nuclectomy depends on the openness or clarity of the nucleus pulposus channels or outlets leading to the bulge. If the channels are closed, which often is the case, evacuation of the nucleus pulposus from the center of the vertebral disc does not affect the bulge and does not provide any benefit to the patient after the percutaneous nuclectomy procedure.

With age, the intervertebral disc continues to lose its ability to retain water, resulting in diminished disc height and narrowed disc space. Disc space narrowing leads to spinal stenosis, which can be painful when nerves are impinged, entrapped or distorted, by the flattened disc or vertebral bone. Depletion of nucleus pulposus from the percutaneous nuclectomy procedure can also cause disc flattening or thinning, leading to vertebral instability and/or spinal stenosis.

Recently, several devices (US Patent No. 5,800,550 to Sertich, 1998; US Patent No. 5,683,394 to Rinner, 1997; US Patent No. 5,423,817 to Lin, 1995; US Patent No. 5,026,373 to Ray et. al., 1991) were designed to fortify the disc space between vertebrae. These types of devices are frequently referred to as spinal cages. Before inserting the device into the disc, the affected disc with portions of vertebral bone above and below the disc are cored out. Usually two holes are cored on each side of the disc for insertion of two spinal cages. Donor bone or bone growth promoting substances are packed into the porous cages. As the vertebrae heal from the coring, new bone grows into and permanently secures the porous cages. The purpose of using spinal cages is to replace the disc and keep the vertebrae apart. However, these vertebrae are permanently fused to each other, without resilient cushion, rotation or mobility.

An improved version of a metallic spinal fusion implant (US patent 5,782,832 to Larsen and Shikhman, 1998) tries to provide both rotational and cushioning capabilities. This invention resembles a disc prosthesis following a complete discectomy. Therefore, at the least, all the complications and postsurgical problems associated with a discectomy also apply when this device is used.

Patent application, PCT/US99/21138, WO 00/40159 by Yeung and Yeung, introduces some devices and methods for fastening herniated and/or bulging discs. The application covers a resiliently bent fastener, screw, suture, staple and tack, with methods to fasten and hold in the bulging annulus.

### Tendon or Ligament Repair

In many accidents or sports related injuries, tendons or ligaments rupture from bones. The traditional repair is to drill through the bone, then pass a suture through to attach the torn tendon back to the bone. Some very strong bone anchors (US patent 5,851,219 to Goble et. al., 1998; and US patent 5,478,353 to Yoon, 1995) have been invented and used with sutures to reattach ruptured tissues. The anchor with its attached suture is inserted into a pre-drilled bone hole. The suture usually comes with a needle for sewing and attaching the torn tissue back to bone. However, suture manipulation requires time, delicate skill from surgeons and surgical space within the patient, which necessitates large and/or multiple incisions with increased risk and recovery time.

### Meniscal Repair

Recently, instead of delivering, manipulating and retrieving sutures to repair tissue in a very tight surgical site, delivery of tacks with barbs (US Patent No. 5,702,462 to Oberlander, 1997; US Patent No. 5,398,861 to Green, 1995; US Patent No. 5,059,206 to Winters, 1991; US Patent No. 4,895,148 to Bays et. aL, 1990; US Patent No. 4,884,572 to Bays et. al., 1989), staples (US Patent No. 5,643,319 to Green et. al., 1997) and fasteners (US Patent No. 5,843,084 to Hart et. aL, 1998; US Patent No. 5,374,268 to Sander, 1994; US Patent No. 5,154,189 to Oberlander et. al., 1992) have been proposed to hold torn tissue through small openings. Unfortunately, very few, if any, of these tacks, staples and fasteners have the holding strength to meet the standard set by sutures.

During the insertion of these devices into tissues, the barbs carve their way into their final holding positions. Unavoidably, the carving damages and weakens the tissue, thereby decreasing the holding strength of the freshly inserted devices. As tension is applied to the fastened tissue, it is not surprising that the barbs can lose their grip, slip and creep along the carved paths created during insertion, leaving gaps in the supposed closure sites. The creeping problem of fastening devices is particularly evident in slow healing tissues, such as menisci, some ligaments and tendons. When gaps are present, the torn tissue does not reattach and heal, even with the passage of time.

Non-biodegradable fasteners often have the problem of device migration, which can be devastating, especially when the device migrates into nerves, joints or vessels, after numerous cycles of tissue remodeling.

In summary, currently most of the tacks and fasteners have one or more of the following 25 drawbacks: (1) weak holding strength, (2) creeping and leaving gaps in the repair site and (3) potential migration into sensitive tissues.

US 2,485,531 (Dzus et al) and W0 99/37219 (Orthodyne, Inc) disclose a surgical toggle bolt and a tissue/bone anchoring system respectively, each having a locking toggle at the leading end thereof.

### SUMMARY OF INVENTION

According to the present invention, there is provided a compression device for treating a bulging disc, herniated disc, intervertebral instability or spinal stenosis as claimed in the appended claims.

All current approaches have one thing in common: tissue removal. Vastly different from the tissue removing procedures, the methods described in the following two sections use techniques and devices to fasten and restrain the bulging, flattened and/or herniated disc to alleviate irritation of the nerve.

For fastening a bulging, herniated or compressed disc, a compression device is used to compress and repair the bulge. The compression device is comprised of a rod-like body, a pivotal toggle, a toggle-retaining nut, a toggle deployment device, a disc restrainer and a restrainer nut. During installation, the compression device is inserted through the defective or dysfunctional disc, using the deployed toggle to distally anchor the compression device. The disc restrainer and the restrainer nut are used to compress and hold back the bulging annulus from impinging upon adjacent nerves, for supporting the annular sidewall or for adding disc height by consolidating annular tissue. Any excess length of rod of the compression device is then removed.

Another disc fastening device described here by way of example only, a tissue fastener, comprises of a rod-like body with two sets 10 of protruding resilient anchors for clamping tissue together. The sets of resilient anchors have opposite clamping directions to counter grip and fasten tissues located at both ends of the tissue fastener. When the tissue fastener is deployed within a bulging disc, the sets of anchors are designed to counter grip or clamp against two tissues, compressing, holding and fastening the tissues together. In the case of the bulging annulus, the tissue fastener compresses, maintains and pinches in the bulge of the annulus. To deliver the fastener, the resilient anchors are compressed or folded within a fastener deployment tube or needle with a plunger located behind the tissue fastener. The deployment tube is inserted through the bulge into the disc, reaching healthy annulus on the other side. The bulge is compressed to a non impinging position by a disc compressor. While the compression is held, the fastener deployment tube is withdrawn while the plunger remains stationary. As the restriction of the deployment tube is lifted, the resilient anchors at each end open, counter fastening, restricting and clamping the annular tissues between two ends of the tissue fastener. Since the anchors are deployed or opened within the compressed annulus, the previously bulging annulus is mechanically anchored, fastened and restrained by the tissue fastener, freeing the adjacent nerve from impingement.

Both the compression device and the fastener are designed to hold, maintain and restrict the bulging annulus. Especially when multiple devices are used, the restrictions fortify the annulus and stabilize the intervertebral disc to minimize pain. Furthermore, the restrictions can also pinch in the annulus, adding disc height and minimizing nerve irritation induced by compressed or flattened discs, a common cause of spinal stenosis.

The tissue fastener and the delivery devices can also be sized and configured to tissues, such as tendons, ligaments, menisci, organs, skin and/or other structures or devices.

Although percutaneous nuclectomy is not a reliable procedure to alleviate low back pain, in some cases, withdrawing nucleus pulposus provides the much needed pain relief without significant risks. One embodiment of this invention implants a disc drain tube to drain the nucleus pulposus from the swollen disc into the abdominal cavity. The disc drain tube has inlet and outlet openings sized to drain the nucleus pulposus when disc pressure is high. To further regulate disc pressure, a pressure-sensing device coupled with a nucleus pulposus discharge gate can be installed within the drain tube.

### REF. NO.

- Bulging Disc: 100
- Nerve Retractor: 101
- Impinged Nerve: 102
- Trocar: 103
- Dilator: 104
- Nut: 105
- Washer: 106
- Toggle: 107
- Rod with Thread: 108
- Toggle Deployment Tube: 109
- Compression Device: 110
- Disc Restrainer: 111
- Restrainer Nut: 112
- Anchor: 113
- Rod-like Tissue Fastener Body: 114
- Lumen of Fastener Deployment Tube: 115
- Penetration Marker: 116
- Plunger: 117
- Disc Compressor: 118
- Normal Functioning Disc: 119
- Tissue Fastener Delivery Device: 120
- Plunger Holding Screw: 121
- Trigger: 122
- Handle: 123
- Disc Drain Tube: 124
- Inlet: 125
- Outlet: 126
- Impingement-Free Nerve: 127
- Nucleus Pulposus: 128
- Pressure-Sensing Device Connector: 129
- Pressure-Sensing Device: 130
- Discharge Gate: 131
- Spring: 132
- Pin: 133
- Hinge: 134
- Meniscus: 135
- Drain Delivery Device: 136
- Humerus: 137
- Tendon: 138
- Torn Tissue: 139
- Bone Hole: 140
- Bolt: 141
- Attachment Slot: 142
- Anchor Hole: 143
- Tissue Fastener: 144
- Tissue Fastener Deployment Tube: 145
- Tissue Manipulative Device: 146
- Tissue Manipulative Element: 147
- Disc Gripping Element: 149
- Restrainer Opening: 149
- Ball: 150
- Ball Coupling: 151
- Ball Joint: 152
- Orientation Line: 153
- Stem of Disc Restrainer: 154
- Tube with Inside Thread: 155
- Disc Restrictor: 156
- Linker: 157
- Link Site: 158
- Vertebral Body: 159

### BRIEF DESCRIPTION OF THE DRAWINGS

Figure 1 depicts a trocar **103** inserted through a bulging disc **100.**
Figure 2 shows the insertion of a dilator **104** through the bulging disc **100.**
Figure 3 indicates a compression device **110** with a toggle deployment tube **109.**
Figure 4 shows the advancement or sliding of the toggle deployment tube **109** moving the toggle **107** from a delivery position to a deployed position.
Figure 5 depicts insertion of the toggle **107** and compression device **110** through the dilator **104.**
Figure 6 indicates the deployment of the toggle **107** outside the vertebral disc by the advancement of the toggle deployment tube **109.**
Figure 7 depicts the installation of a disc restrainer **111** and a restrainer nut **112** on the threaded rod **108** of the compression device **110.**
Figure 8 shows the tightening of the restrainer nut **112** and the disc restrainer **111** to press in the bulging disc.
Figure 9 depicts the result of cutting the excess threaded rod **108.**
Figure 10 indicates a depression in a disc restrainer **111** for concealing the restrainer nut **112.**
Figure 11 indicates a bolt **141** for tightening the disc restrainer **111** onto a tube **155** with inside thread, connecting to the toggle **107** assembly.
Figure 12 shows a mid-longitudinal view of two vertebral bodies **159** sandwiching a flattened or compressed disc **100.**
Figure 13 depicts a mid-longitudinal view of a thickened intervertebral disc **100,** induced by the compression device **110,** further separating the two vertebral bodies **159.**
Figure 14 shows a pair of toggles **107** linked by a hinge **134.**
Figure 15 depicts a toggle **107** pivoting around a pin **133.**
Figure 16 shows a disc restrictor **156** equipped with two disc restrainers **111** individually bolted onto a tube **155** with inside thread.
Figure 17 indicates a compressed or bulging disc **100** being fastened, supported and/or thickened by two disc restrictors **156.**
Figure 18 depicts a tissue fastener **144** with anchors **113** resiliently deployed to counter grip tissues in their preferred or biased positions.
Figure 19 shows the tissue fastener **144** inside a tissue fastener deployment tube **145** with the anchors **113** resiliently folded or compressed in their delivery or closed positions.
Figure 20 depicts the insertion into a bulging disc **100** with the folded tissue fastener **144** and a plunger **117** within the fastener deployment tube **145.**
Figure 21 indicates the compression of the bulging disc **100** by a disc compressor **118.**
Figure 22 shows the deployment of the tissue fastener **144** by withdrawing the fastener deployment tube **145** while the disc compressor **118** presses down on the bulge and the plunger **117** remains stationary.
Figure 23 depicts a fully deployed tissue fastener **144** with extended anchors **113** holding and maintaining the compressed position of the annulus **119.**
Figure 24 shows a tissue fastener delivery device **120** for delivering a tissue fastener **144.**
Figure 25 depicts the tissue fastener delivery device **120** following the deployment of the tissue fastener **144.**
Figure 26 indicates a sharpened tissue fastener tube **145** for use as a needle for tissue puncturing.
Figure 27 shows a cross-section of the mid-longitudinal view of the attachment between the plunger **117** and the tissue fastener **144** through a linker **157** on the plunger **117** onto a link site **158** of the tissue fastener **144.**
Figure 28 indicates a partially withdrawn tissue fastener deployment needle **145** deploying the distal anchors **113** of the fastener **144** into the bulging disc **100.**
Figure 29 depicts the inward pulling of the distal bulging 100 wall by the plunger 117 connected to the tissue fastener **144.**
Figure 30 shows compression of the proximal bulging **100** wall by the disc compressor **118.**
Figure 31 indicates the complete deployment of the tissue fastener **144** by fully withdrawing the tissue fastener deployment needle **145** from the bulging disc **100.**
Figure 32 depicts the tissue fastener **144** disconnected from the plunger, counter gripping both distal and proximal annular tissues, thereby holding in the bulging disc **100.**
Figure 33 shows two tissue fasteners **144** holding, supporting, strengthening and/or thickening the sidewall of the bulging disc **100.**
Figure 34 indicates a well supported and thickened intervertebral disc **100,** maintained and supported by two compression devices **110** and a tissue fastener **144.**
Figure 35 shows a tissue fastener **144** with a spring **132** for a tight and added elastic fastening.
Figure 36 depicts anchors **113** of a tissue fastener **144** restricted within an elliptical fastener deployment tube **145,** equipped with a plunger **117.**
Figure 37 shows a disc compressor **118** and a disc restrainer **111** containing a stem **154** with two attachment slots **142** fitted over the fastener deployment tube **145.**
Figure 38 depicts the fastening of the disc restrainer **111** by the deployed anchors **113** of the tissue fastener **144** after the removal of the fastener deployment tube **145.**
Figure 39 depicts a combination of a tissue fastener **144** with a disc restrainer **111** to repair a bulging disc.
Figure 40 indicates a combination of compression device **110** and a tissue fastener **144** to repair a bulging disc.
Figure 41 depicts a pair of wide anchors **113** for holding soft and fragile tissue.
Figure 42 indicates a pair of sharp anchors **113** for piercing and trapping tissue.
Figure 43 shows a pair of sharp and wide combination anchors **113** for penetrating then grasping and gripping tissue.
Figure 44 depicts a tissue fastener **144** with some deployed anchors **113** perpendicularly extended and closely packed to each other.
Figure 45 depicts a tissue fastener rod **114** with an anchor hole **143.**
Figure 46 indicates a pair of modular anchors **113** fitted into the anchor hole **143.**
Figure 47 shows an end view of a tissue fastener **144** with deployed anchors **113** aligned within the same plane.
Figure 48 depicts an end view of a tissue fastener **144** with deployed anchors **113** perpendicularly extended or oriented from each other.
Figure 49 indicates an end view of a tissue fastener **144** with deployed anchors **113** offset from each other.
Figure 50 shows a curved fastener deployment needle **145** with an orientation line **153** and penetration markers **116.** A flexible tissue fastener **144** and plunger **117** are within the needle **145.**
Figure 51 indicates a cross-section of a fastener **144** and a fastener deployment tube **145** with a widened lumen **115** to prevent fastener **144** rotation within the lumen **115.**
Figure 52 depicts a mid-longitudinal view of a fastener rod **114** equipped with a ball joint **152,** formed by a ball **150** connected to a ball coupling **151.**
Figure 53 shows a disc drain tube **124** with nucleus pulposus inlets **125** and an outlet **126.**
Figure 54 depicts the insertion of a disc drain tube **124** into a bulging disc **100.**
Figure 55 indicates the draining of the nucleus pulposus **128** by the disc drain tube **124** resulting in a non-bulging disc **119.**
Figure 56 shows a disc drain tube **124** with a nucleus pulposus pressure sensing device **130** and discharge gate **131.**
Figure 57 depicts a compression device **110** with nucleus pulposus draining capability.
Figure 58 indicates the insertion of a tissue fastening deployment tube **145** with a tissue fastener **144** through a torn tendon **138** into a bone hole **140.**
Figure 59 shows a tissue-manipulating device **146** positioning the torn tendon **138** back over the bone hole **140.**
Figure 60 depicts a fully deployed tissue fastener **144** fastening the torn tendon **138** back onto the bone.
Figure 61 shows a tissue fastener **144** holding a torn **139** portion of a meniscus **135.**

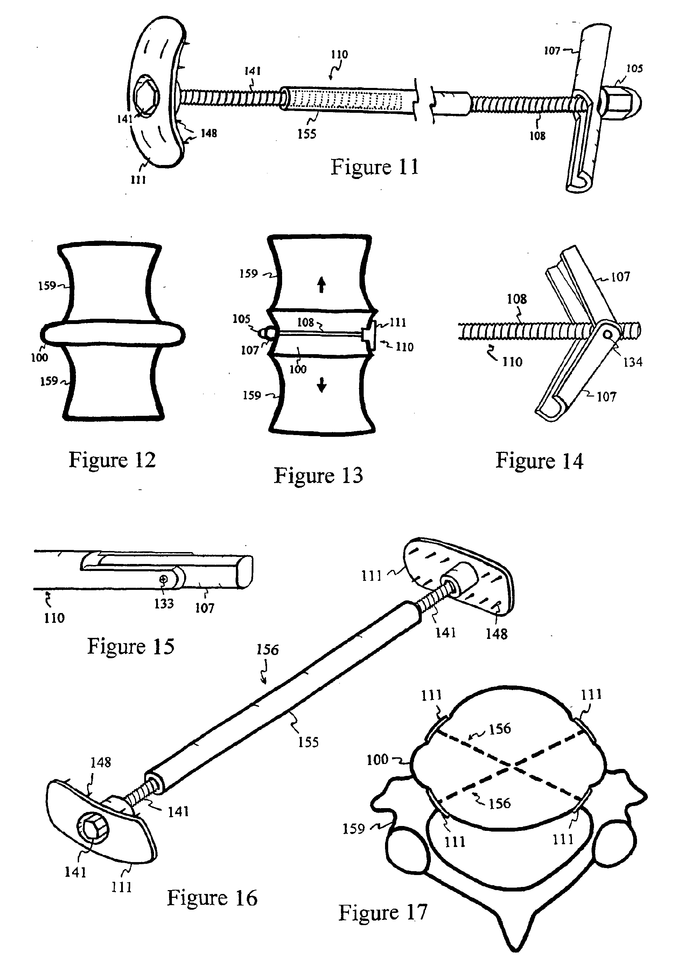

Figures 16-61 do not form part of the invention and are described by way of example only.

### DETAILED DESCRIPTION OF THE EMBODIMENTS

### Low Back Pain

Low back pain from bulging, herniated or compressed discs **100** is one of the most common, prevalent, costly, painful and debilitating ailments afflicting mankind. As mentioned, treatments ranging from traditional to percutaneous approaches all have their drawbacks; some are very serious.

All current approaches have one thing in common: tissue removal. Vastly different from the tissue removing procedures, the methods described in the following two sections use techniques and devices to fasten and restrain the bulging, flattened and/or herniated disc **100** to alleviate irritation of the nerve **102.**

### Compression Device, Disc Restrictor and Methods for Restraining Bulging Discs

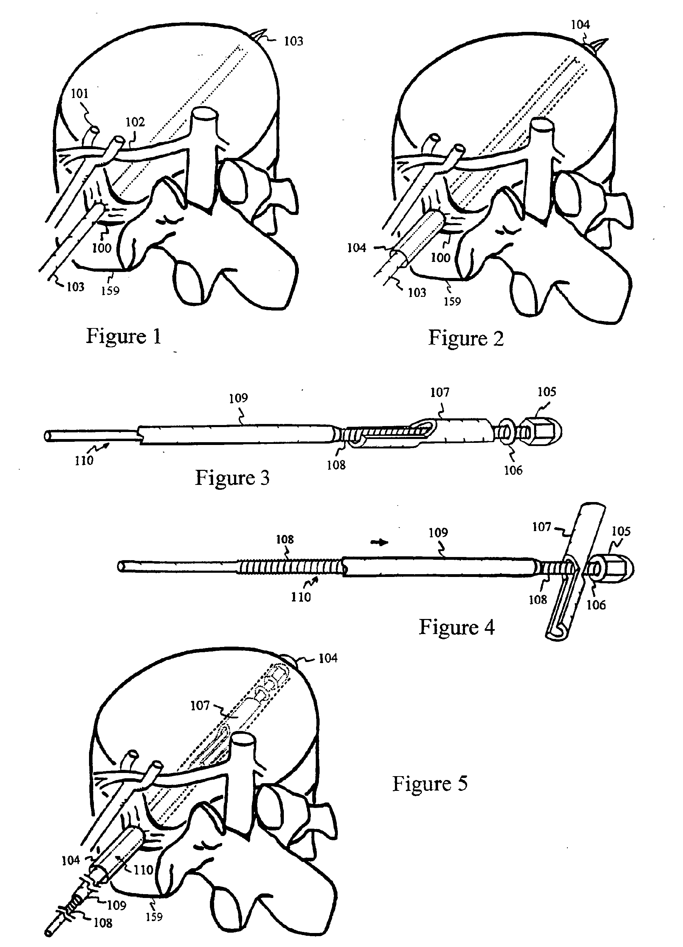

Placement of the compression device **110** begins with a trocar **103** guided through the bulge into the intervertebral disc **100,** as depicted in Figure 1. A laminotomy, removal of a small portion of laminal bone, may be necessary to access the bulge. Numerous existing guiding techniques, such as anteroposterior and lateral fluoroscopy, MRI, ultrasound or others can be used to guide the trocar **103** into place. To avoid injury of blood vessels, such as the common iliac artery or vein, located anterior to the intervertebral disc **100,** an angiogram of the blood vessels can be mapped out prior to trocar **103** insertion. Following the trocar **103,** a dilator **104** is inserted into the bulging disc **100,** as shown in Figure 2. The trocar **103** is then removed.

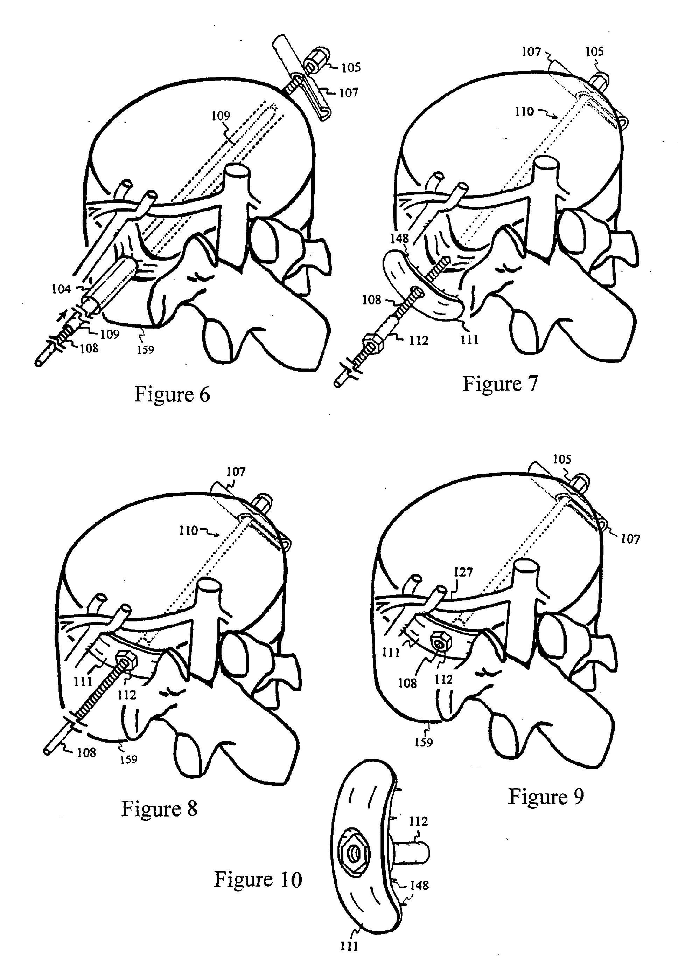

Figure 3 depicts a compression device **110** with a threaded rod **108** as the body, a nut **105** and a washer **106** to hold a toggle **107,** and a deployment tube **109** to activate the toggle **107.** The unique tubular toggle **107** on the compression device **110** is designed to provide high tensile strength and conform within a small cylindrical space within the dilator **104.** The toggle **107** has two semi-cylindrical sections centrally connected with openings facing opposite directions. In a delivery position, the concave sides of the opposing semi-cylindrical sections partly cover the rod **108;** as depicted in Figure 3, the toggle **107** aligns parallel and fits over the threaded rod **108** of the compression device **110.** In a deployed position, the toggle **107** rotates or opens, forming a T-like configuration with the rod **108** of the compression device **110,** as shown in Figure 4. In the delivery position, the compression device **110** is inserted into the dilator **104** as indicated in Figure 5. The toggle **107** is deployed outside the annulus by a gentle push of the sliding toggle deployment tube **109,** as indicated in Figure 6, to secure the compression device **110** at the distal end. Both the deployment tube **109** and the dilator **104** are withdrawn from the bulging disc **100.** A disc restrainer **111** and a restrainer nut **112** are installed on the threaded rod **108** as depicted in Figure 7.

To prevent possible movement of the compression device **110,** especially on the weakened bulging annulus, disc gripping elements **148** on the disc restrainer **111,** as shown in Figures 7 and 10, are designed to grasp, hold and/or bundle the weakened annulus together during compression of the bulging disc **100.** The bulge is compressed by the disc restrainer **111** and the restrainer nut **112,** away from the adjacent, previously impinged nerve, as depicted in Figure 8. The disc restrainer **111** can be made of a resilient or elastic material, such as nickel-titanium alloy or tempered stainless steel spring, to snugly compress and conform to the contour of a normally shaped annulus with minimal protrusion.

Excess threaded rod **108** is cut by a pair of endoscopic pliers (not shown); the result is shown in Figure 9. Figure 10 shows a depression or a recessed pocket in a disc restrainer **111** for concealing the restrainer nut **112** to minimize the possibility of neural impingement by a protruding restrainer nut **112.** It is also possible to eliminate the cutting of the threaded rod **108** by substituting the restrainer nut **112** with a bolt **141.** In Figure 11, the bolt **141** passes through a depression or a recessed pocket of the disc restrainer **111** into a tube **155** with inside thread, connected to the toggle **107** assembly. As the bolt **141** advances into the tube **155** and toggle **107** assembly, the disc restrainer **111** is tightened, restricted, compressed and fastened into the annulus.

In addition to alleviating nerve **102** impingement by repositioning the bulging annulus **100,** the compression and tightening of the bulging disc **100** by the restrainer **111** can collapse and seal channels of the leaking nucleus pulposus **128,** thus stopping the herniation.

Back pain caused by a flattened, bulging or compressed disc **100** may lead to intervertebral instability or spinal stenosis. The intervertebral instability resembles an out-of-control car riding on one or more flat tires with deflated and unsupported sidewalls. A flattened intervertebral disc **100** causes swaying between vertebral bodies, leading to pain in surrounding ligaments and facet joints. Figure 12 shows a mid-longitudinal view of a flattened bulging disc **100** between two vertebral bodies **159.** Figure 13 indicates the compression-induced support and thickening of the previously bulging disc **100** by the compression device **110.** The compression fortifies and supports the sidewall of the annulus from rolling, thereby minimizing vertebral instability. The bulging and flattened disc **100** is being compressed and consolidated, thereby adding cushioning annulus to build height between the two vertebrae **159.** The main functions of the compression device **110** are to fasten, restrict, tighten, support, fortify, maintain and/or pinch in the annulus of a bulging or flattened disc **100** to alleviate nerve **102** impingement and minimize intervertebral instability and spinal stenosis.

Other forms of toggles **107** can also be used to fasten bulging or herniated intervertebral discs **100.** A two-pieced toggle **107** linked by a hinge **134** connected by a pin is shown in Figure 14. Figure 15 depicts a pivotal toggle **107** rotating on another type of pin **133** or screw.

To improve the strength of disc fastening, two disc restrainers **111** can be individually tightened by two bolts **141** to restrict the bulging of an intervertebral disc **100.** The modified device is called a disc restrictor **156,** as shown in Figure 16. To install the disc restrictor **156,** it is likely that both abdominal and posterior incisions are required, followed by trocar **103** and dilator **104** insertions.

Figure 17 depicts two disc restrictors **156** fastening, tightening, restricting, supporting and/or pinching in four sections of annulus of a bulging disc **100,** adding sidewall support to minimize intervertebral instability and/or building disc height to minimize spinal stenosis in four sections of the bulging and/or flattened disc **100.** Similarly, multiple compression devices **110** can also be guided and installed through posterior incisions to minimize back pain and repair dysfunctional intervertebral disc **100.**

The compression device **110** or the disc restrictor **156** is well suited for fastening a lateral bulging annulus without excessively encroaching upon the anterior and posterior longitudinal ligaments and without protruding into the spinal canal. The angle of trocar **103** penetration is directed posterior to the common iliac vessels or inferior vena cava. Surgical placement of the compression device **110** can also be done anteriorly through the abdomen to avoid injury to the blood vessels.

To further avoid blood vessels or vertebral bone, the trocar **103** and the dilator **104** can be made with curvatures. To accommodate the curvature of the dilator **104,** the compression device **110** can be made with flexible material.

Components of the compression device **110** or the disc restrictor **156** can be made with material such as titanium, nickel-titanium, stainless steel or other metals or alloys. Some relatively new and biocompatible polymers, such as poly-ether-ether-ketone, DELRIN (acetal resin), polysulfone, polycarbonate, polypropylene, polyethylene or others, may have sufficient strength and durability. Especially for testing purposes, a biodegradable compression device **110** or a portion of the device can be made with poly-lactate, poly-glycolic or other biodegradable polymers. All materials should be able to withstand sterilization by gamma, electron beam, ETO or steam to prevent infection.

The compression device **110** and the disc restrictor **156** can also be coated or blended with radiopaque, echogenic, growth factor, analgesic, sealing, blood clotting, anti-biotic and/or other materials.

Unlike microdiscectomy or percutaneous nuclectomy, the compression device **110** and/or the disc restrictor **156** preserves disc material and provides direct, predictable and durable repair of the defective or dysfunctional disc **100** with the potential to stop further leakage of nucleus pulposus. Disc fastening also provides the possibilities of minimizing intervertebral instability and increasing disc height to relieve pain and/or discomfort.

### Tissue Fastening Devices and Methods for Fastening Bulging Discs

Another intervertebral disc fastening device called a tissue fastener **144** is designed to function while embedded entirely within the repaired disc **119.** Three major benefits of using the tissue fastener **144** are (1) minimal possibility of rupturing major blood vessels since it does not penetrate through the anterior side of the dysfunctional disc **100,** (2) dissection of anterior and posterior longitudinal ligaments is not necessary and (3) no part protrudes to impinge nerve **102,** spinal cord or blood vessels.

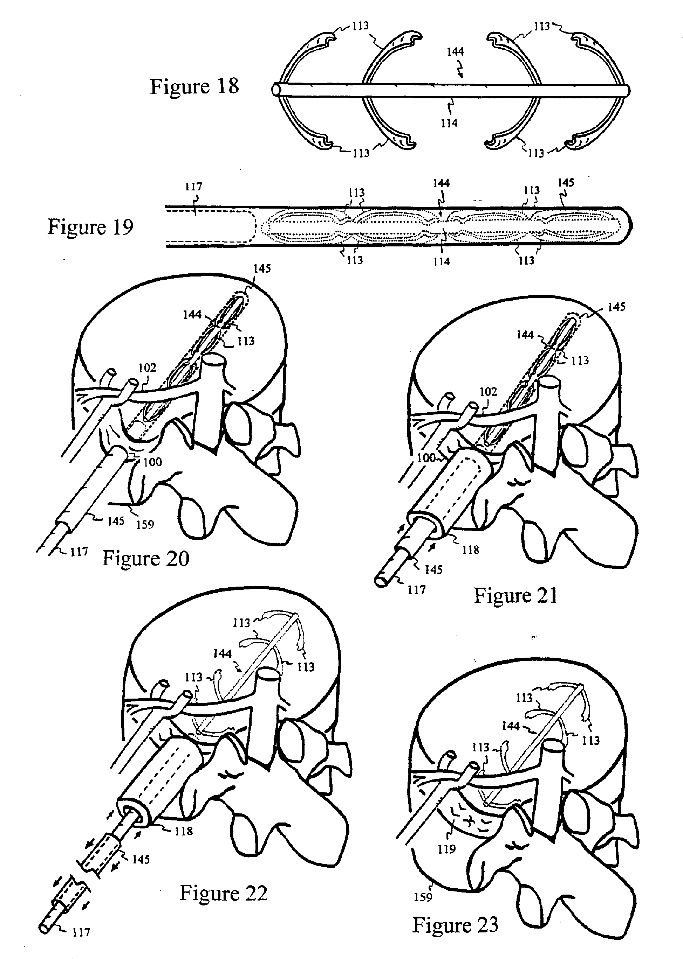

Figure 18 depicts a tissue fastener **144** with a rod **114** as body and resilient anchors **113** as tissue clamping elements in their deployed positions. The anchors **113** or clamping elements are made with elastic or shape memory material, biased toward the open or deployed positions. The anchors **113,** with distinct gripping directions, are designed to counter clamp, grip, fasten and hold tissues together, resisting pullout from the tissue. The rod **114** joins the anchors **113** and provides the tensile strength required to hold two tissues together. The resilient anchors **113** are designed to be elastically or resiliently folded or compressed within a fastener deployment tube **145,** as depicted in Figure 19, for delivery into a bulging disc **100.** The folded spring-like anchors **113** are in delivery positions in the fastener deployment tube **145.**

To install the tissue fastener **144,** a trocar **103** is guided through the bulge **100** into the intervertebral disc with anteroposterior and lateral fluoroscopy, MRI, ultrasound or another method. Unlike Figure 1, the trocar is preferred not to penetrate through the intervertebral disc **100,** thus avoiding the possibility of puncturing blood vessels. Following the trocar **103,** a dilator **104** is inserted into the disc **100** to enlarge the opening.

The fastener deployment tube **145,** loaded with a tissue fastener **144,** is inserted into a bulging disc **100,** as indicated in Figure 20. A disc compressor **118** or tissue manipulative device is used to press the bulge back to a non-nerve-impinging position, as depicted in Figure 21. While the compressor **118** is pressing back the bulge and the plunger **117** is held stationary, the fastener deployment tube **145** is withdrawn to deploy the tissue fastener **144** within the compressed intervertebral disc, as shown in Figure 22. Since the resilient anchors **113** are made of elastic or shape memory material, when the restriction of the fastener deployment tube **145** is lifted, the resilient anchors **113** extend outward to their deployed positions. The anchors **113** at the distal end of the tissue fastener **144** grip and fasten onto the healthy annulus. The anchors **113** at the proximal end of the fastener **144** open to clamp, grip, fasten and hold the compressed annulus in place. In essence, the deployed tissue fastener **144** locks and maintains the compressed, previously bulging annulus in a non-nerve-impinging position, forming a repaired disc **119,** as shown in Figure 23. At the same time, the compression and fastening of the bulging annulus can, and likely will, collapse and seal the channels of the leaky nucleus pulposus **128** near the bulging annulus, stopping the herniation.

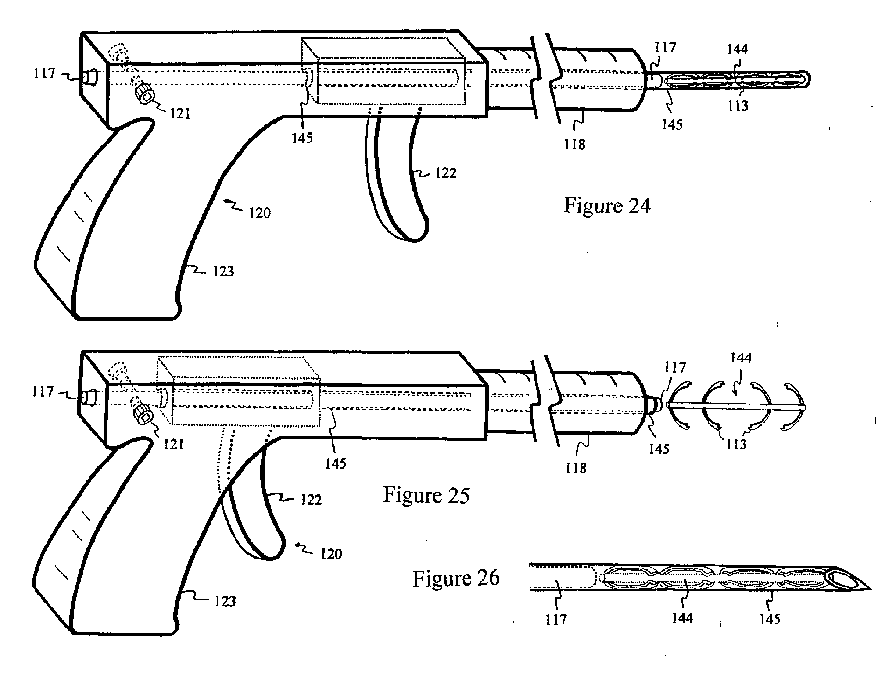

For the surgeon's convenience, inserting the fastener deployment tube **145,** compressing the bulging disc **100** and deploying the tissue fastener **144** can all be done with one hand. Figure 24 shows a tissue fastener delivery device **120** with a tissue fastener **144** in the lumen of fastener deployment tube **145** operated by a trigger **122,** a stationary plunger **117** held by plunger screws **121** and a built-in disc compressor **118** attached to the device handle **123.** A trigger lock can be added to prevent the accidental release of the tissue fastener **144** during device **120** insertion. Figure 25 shows the deployment of the tissue fastener **144** from the tissue fastener delivery device **120** by using the trigger **122** to withdraw the fastener deployment tube **145.** With the restriction of the deployment tube 145 removed, the anchors **113** of the tissue fastener **144** extend to their preferred deployed positions. It is also possible to load multiple tissue fasteners **144** within the fastener deployment tube **145** with the capability of deploying one fastener **144** at a time, facilitated by increments in trigger **122** pulling. Multiple fasteners **144** can be used to anchor defective tissue, such as the intervertebral disc or others, in place. The distal end of the fastener deployment tube **145** can be sharpened, as indicated in Figure 26, to facilitate the puncturing of the bulging disc 100. The deployment tube or needle **145** can also be coated with radiopaque, echogenic, lubricant or other coating material to facilitate device insertion.

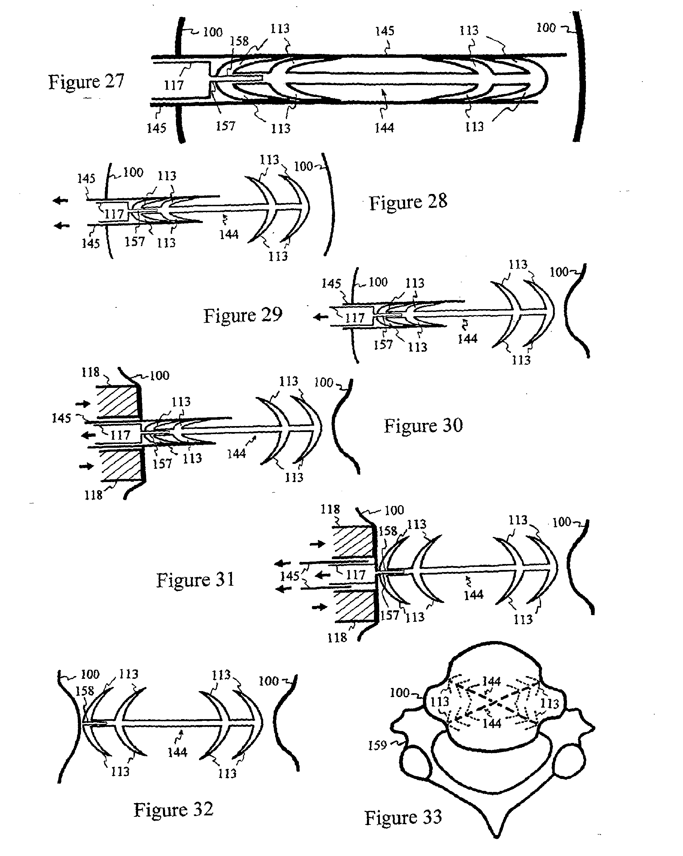

For extra tight tissue fastening of the bulging disc **100,** a linkage may be made between the plunger and the fastener **144** to provide pulling capability upon the annulus by the partially deployed fastener **144.** Figure 27 depicts a mid-longitudinal view of a screw-like linker **157** protruding from the distal end of the plunger **117** and fastening into an inside threaded link site **158** in the tissue fastener **144.** The linked assembly, located within a tissue fastener deployment needle **145,** is then inserted into a bulging disc **100.** Figure 28 indicates a mid-longitudinal view of deployed distal anchors **113** fastening onto the distal annulus of the bulging disc **100** by partially withdrawing the tissue fastener deployment needle **145,** while holding the plunger **117** stationary. Figure 29 depicts the pulling of the plunger **117** connected to the tissue fastener **144,** pulling in the distal bulging annulus **100.** Figure 30 shows the compression of the proximal bulging disc **100** by the disc compressor **118,** while the plunger **117** is continuously being pulled. Figure 31 depicts the full deployment of the tissue fastener **144** by withdrawing the remaining portion of the tissue fastener deployment needle **145** from the bulging disc **100,** while continuing to compress the disc **100** and pull the plunger **117.** After disconnecting the linker **157** of the plunger **117** from the link site **158** of the fastener **144,** Figure 32 shows the fastener **144** tightly fastening the previously bulging disc **100** by counter gripping, clamping, holding, supporting and pulling in the annular wall with the anchors **113.**

Other connecting linkages between the tissue fastener **144** and the delivery device **120** are possible. For example, a screw from the proximal portion of the tissue fastener **144** can be inserted into a screw hole on the plunger **117.** A latch or a hinge can couple with an indentation to link between the fastener **144** and the plunger **117,** or between the fastener **144** and the tissue deployment needle **145.**

In addition to disc fastening using compression devices **110** in Figure 13 or disc restrictors **156** in Figure 17, other disc fastening devices, such as the tissue fasteners **144,** can also minimize intervertebral instability and/or thicken intervertebral disc space to alleviate pain and discomfort. Figure 33 depicts the result of two counter gripping tissue fasteners **144** deployed by the method described in Figures 27 to 32 to support, strengthen, restrict and/or pull in the annular wall of the bulging disc **100.**

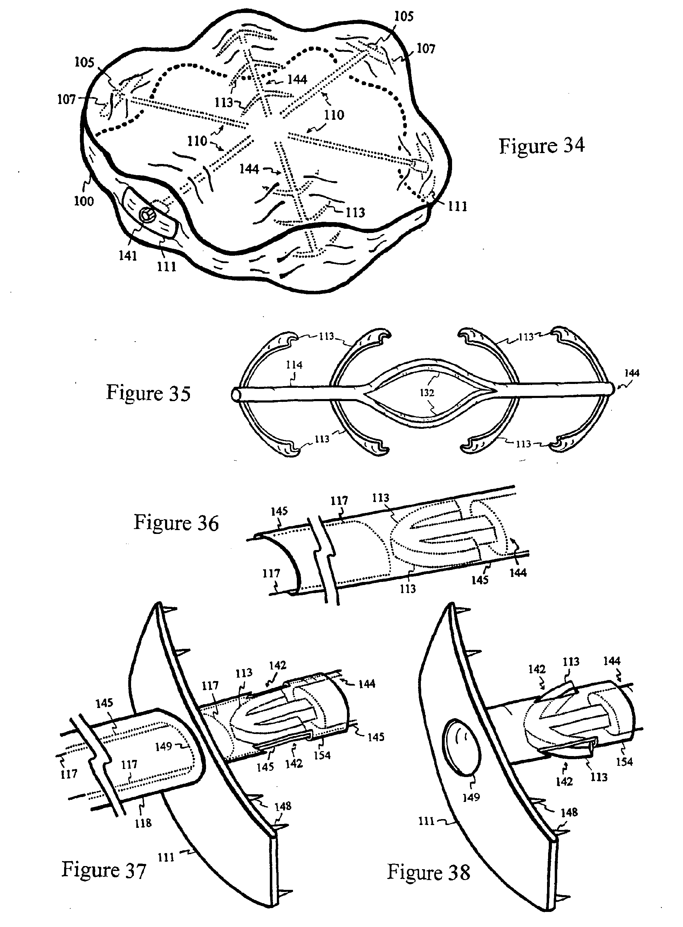

Figure 34 shows a previously flattened disc **100** repaired by the combination of two compression devices **110** and a tissue fastener **144** to build disc height in multiple sections of the disc **100** to treat spinal stenosis. At the same time, the flattened annulus is well supported to minimize intervertebral instability. Figure 34 also indicates that the tissue fastener **144** can be well suited for fastening the mid-section of the compressed disc **100** by holding or restricting the anterior and posterior portions of the annular wall with no device protrusion in the central canal.

The tissue fastener **144** with an elastic spring **132,** as indicated in Figure 35, is particularly well suited for restricting and strengthening the bulging disc **100.** As the tissue fastener deployment tube **145** is partially withdrawn, the distal anchors **113** extend out to make initial penetration into tissue. As the tissue fastener deployment tube **145** continues to be withdrawn, the deployment of the spring **132** follows. As the spring **132** is freed from the tubular **145** restriction, the spring **132** resiliently spreads apart in the gelatinous nucleus pulposus, pulling in the distal portion of the tissue fastener **144.** The pulling of the fastener **144** facilitates the deployment of the distal anchors **113,** securing the clamping positions within the tissue, before the subsequent deployment of proximal anchors **113.** It is also possible to link the spring **132** loaded fastener **144** to the plunger **117** for additional manipulation. In essence, the spring loaded tissue fastener **144** provides tight and elastic tissue fastening.

For holding and fastening degenerated or badly damaged annular tissue, the anchors **113** of the tissue fastener **144** can be utilized to fasten a disc restrainer **111.** Figure 36 depicts anchors **113** of a tissue fastener **144** restricted in an elliptical fastener deployment tube **145** equipped with a plunger **117.** A disc restrainer **111** having an elliptical opening **149** and a stem **154** with attachment slots **142** is fitted over the elliptical fastener deployment tube **145** along with a disc compressor **118,** as shown in Figure 37. Alignment of the attachment slots **142** over the deployed anchors **113** is facilitated by the non-rotary elliptical stem **154** over the elliptical fastener deployment tube **145.** The assembly is inserted into the bulging disc **100.** The disc compressor **118** is used to press the disc restrainer **111** against the bulge to a non-nerve-impinging position. While the disc compression continues and the plunger **117** is held stationary behind the tissue fastener **144,** the elliptical fastener deployment tube **145** is withdrawn. As the restriction of the deployment tube **145** is removed, the resilient anchors **113** open and fasten into the adjacent attachment slots **142** in the stem **154** of the restrainer **111,** as indicated in Figure 38. The anchors **113** at the distal end of the tissue fastener **144** fasten onto healthy annulus, providing the fastening strength for the disc restrainer **111** to compress the bulging tissue.

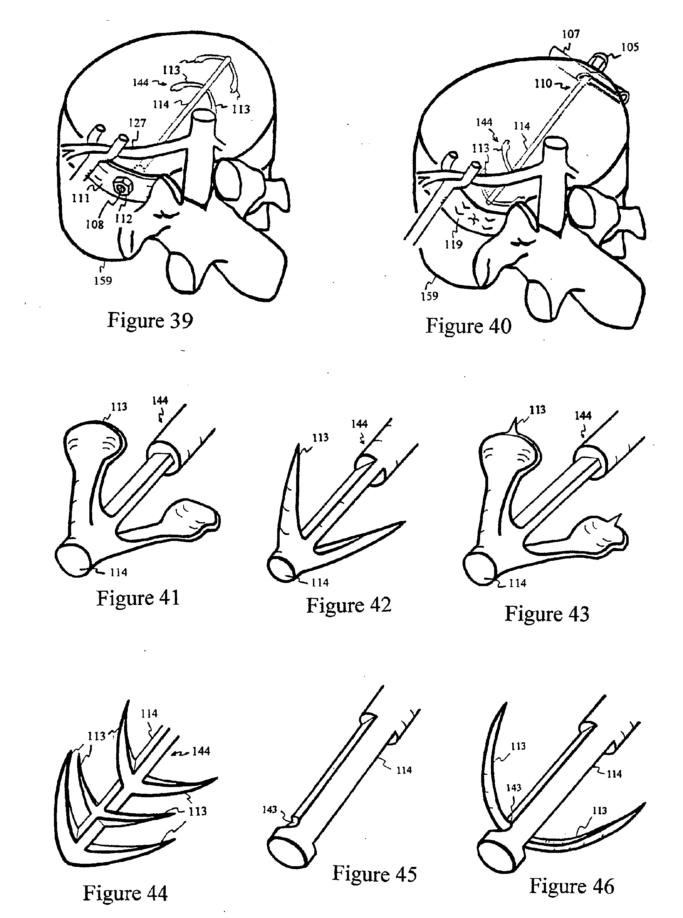

It is also possible to have a combination of a tissue fastener **144** with a compression device **110** to repair a dysfunctional disc **100.** Figure 39 depicts a bulging disc **100** repaired by resilient anchors **113** at the distal end and a disc restrainer **111** with a restrainer nut **112** at the proximal end. This combination minimizes the possibility of injuring blood vessels anterior to the intervertebral disc and maximizes the restraint of a disintegrating bulging and/or herniated disc **100** posteriorly. The tissue fastener **144** and disc restrainer **111** combination can be made with a partially threaded rod **114** fitted within a tissue fastener deployment tube **145.** After the anchors **113** are deployed by the withdrawal of the deployment tube **145,** a disc restrainer **111** and a restrainer nut **112** are installed. Excess threaded rod **114** is cut; the result is shown in Figure 39. The disc restrainer **111** can also be connected to the tissue fastener **144** by anchors **113** as shown in Figures 37 and 38. The tissue fastener **144** can also link to the plunger **117** to provide pulling and manipulative capabilities to the fastener **144.**

Figure 40 shows a bulging disc **100** repaired by a compression device **110** at the distal end and tissue fastener **144** at the proximal end of another combination device. This combination provides a strong toggle fastening combined with disc repair without protrusions near the nerve or spinal cord. The combination is best suited for disc bulges **100** at or near the central canal. The concealed anchors **113** fasten and hold the previously bulging annulus **119** with minimal disturbances to the spinal cord and posterior longitudinal ligaments. The combination of compression device **110** and tissue fastener **144** can be achieved with a toggle deployment tube **109,** which also serves as a tissue fastener deployment tube **145.** A detachable linkage, similar to the one shown in Figure 27, may be used to connect the fastener **144** to the plunger **117** of a delivery device. The procedure for inserting and deploying the toggle **107** is similar to Figures 1, 2, 5 and 6. After the toggle **107** is deployed, the fastener-linked plunger **117** is pulled to secure the distal end of the combination device. A disc compressor **118** is then pressed into the bulging disc 100 and the toggle deployment tube **109** or the fastener deployment tube **145** is withdrawn to deploy the anchors **112.** Similar procedures are shown in Figures 21 and 22. The linkage is then disconnected; and the result is a normal functioning disc **119,** as shown in Figure 40, fastened by the combination devices.

One of the most important mechanisms in tissue anchoring by the fastener **144** is the initial tissue penetration. After withdrawing the tissue fastener deployment tube or needle **145,** some anchor **113** penetration into the surrounding tissue during the anchor **113** deployment must be initiated. As the anchors **113** are pulled against the tissue, the anchors **113** flare open within the tissue, reaching the maximum anchoring power to firmly hook, clamp, trap and fasten the tissue. Therefore, the tip and/or the outer edge of the anchor **113** should have some tissue penetrating capabilities to initiate entry into the tissue, especially a tough tissue like the annulus. On the other hand, the inner edge of the anchor **113** is responsible for fastening, holding, clamping and trapping the tissue; therefore, it should be made as wide and as dull as possible to prevent cutting the tissue, thus maximizing the fastening strength of the tissue fastener **144.**

The shape, stacking/orientation, spacing and numbers of the anchor **113** can influent the fastening strength of the tissue fastener **144.** To maximize tissue-anchoring power, the shape of the anchors **113** on the tissue fastener **144** can make a significant difference. Figure 41 depicts a pair of wide anchors **113**, which may provide effective anchoring in soft and easily ripped tissue, such as skin, lung, liver or other organs. Figure 42 shows a pair of sharp anchors **113,** which may be good for anchoring tough, fairly dense and hard to rip tissue, such as bone or the annulus of intervertebral discs. The sharp anchors **113** open as a pair of hooks, catching and trapping the anchored tissue at the bases of the sharp anchors **113.** Figure 43 indicates a pair of combination sharp and wide anchors **113.** Within the tissue, the sharp tips can assist the wide anchors **113** to initiate tissue penetration or a foothold, allowing the full deployment of the wide anchor **113** within the tissue. The combination may provide the maximum anchoring power without ripping tissue.

Additional anchors **113** may improve fastening strength of the tissue fastener **144.** Figure 44 depicts the anchors **113** rotated between levels, allowing more anchors **113** to be densely stacked within a given length of the tissue fastener **144.** As more anchors **113** are available, more layers of tissue will be fastened to further improve the fastening strength within a small space. -

Many other factors, including the length, size, and resiliency of the anchors **113** and the rod-like body **114,** also contribute to overall fastening strength and effectiveness of tissue fastening.

The composition of the tissue fastener **144** can be formed from modular parts. Figure 45 depicts an anchor hole **143** in the rod **114** of a tissue fastener **144.** Figure 46 indicates a modular anchor **113** inserted into the anchor hole **143.**

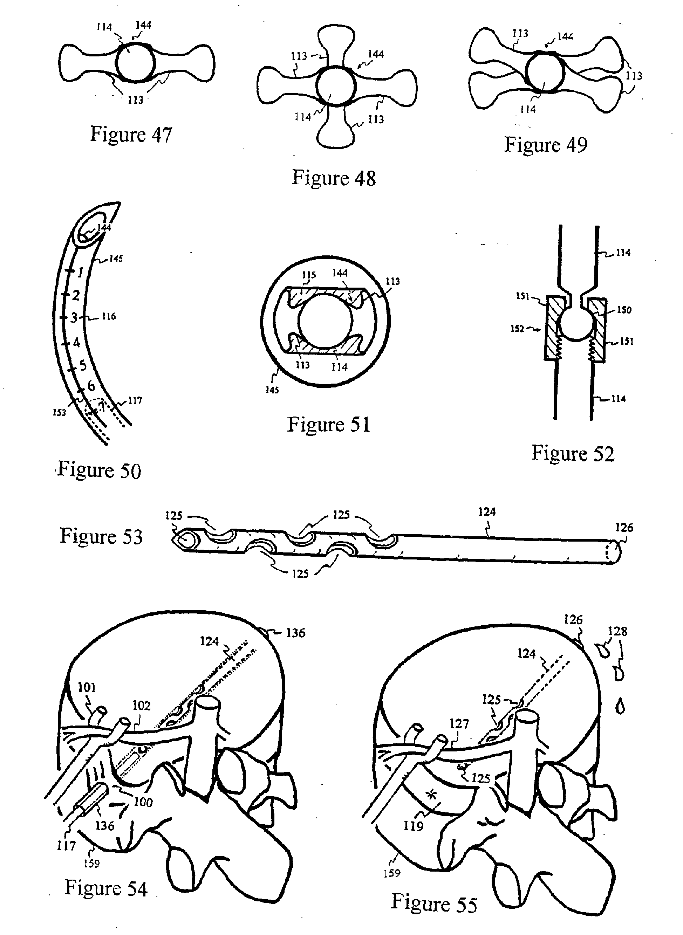

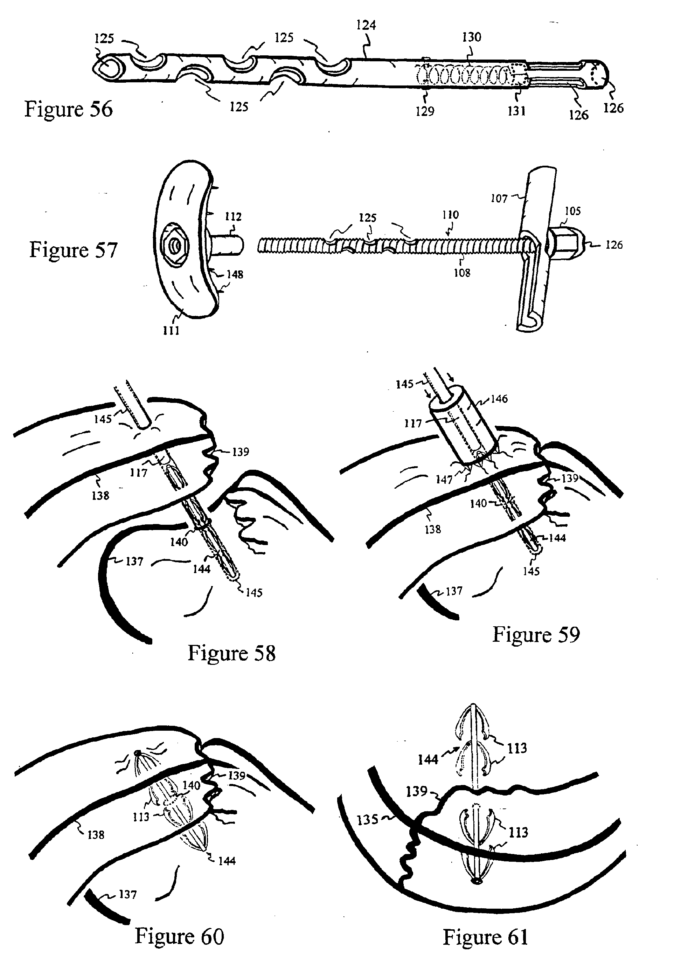

The alignment and/or the stacking of the anchors **113** can be customized, specifically for each type of tissue. The end view of the fastener **144** in Figure 18 is depicted in Figure 47, with the set of deployed anchors **113** aligned together to fasten onto tissue. This type of anchor **113** alignment is good for fastening thin tissue by minimizing the protrusion of the deployed anchors **113.** Tissue fasteners **144** with anchors **113** aligned together, as indicated in Figure 47, may be ideal for repairing meniscal tears **139,** as shown in Figure 61, since the meniscus **135** is a thin cartilage between delicate and sensitive femorotibial joint tissues. Figure 48 depicts the end view of deployed anchors **113** similar to the ones in the fastener **144** shown in Figure 44. The anchors **113** are stacked over each other at right angles to hold large amounts and the most layers of surrounding tissue. This type of anchor **113** alignment, indicated in Figures 48 and 44, should provide the most anchoring power, which may be suitable for fastening a tendon **138** to bone. Figure 49 depicts the end view of a tissue fastener **114** with the top set of deployed anchors **113** slightly rotated from the set below, to provide some improved anchoring power over the one shown in Figure 47. This type of anchor **113** alignment may be useful for fastening bulging discs **100** without excessive protrusion towards the sensitive vertebral end plates, but at the same time occupying and anchoring the maximum amount of annulus to ensure a long lasting disc **100** repair.

The fastener deployment tube **145** can also be made with a curvature, as shown in Figure 50, to access a hard to reach bulge. To accommodate the curvature of the deployment tube **145,** the rod **114** of the tissue fastener **144** and the plunger **117** should be made flexible or curved.

The orientation of the tissue fastener **144** may play an important role in the patient's comfort after the device has been implanted. It probably would not be comfortable to have the anchors **113** within the disc, pointing toward the end plates of both vertebral bodies **159** where pain sensation can be felt. To avoid such post-surgical discomfort, the tissue fastener **144** is loaded and oriented so that the anchors **113** are aligned with a visible orientation line **153** drawn on the fastener deployment tube **145,** as indicated in Figure 50. Similarly, the tissue fastener **144** can be loaded in a designated orientation with respect to the handle **123** of the delivery device **120.** To ensure proper fastener **144** alignment relative to the orientation line **153** or the handle **123,** the inside lumen **115** or chamber of the fastener deployment tube **145** can be widened or made non-round to prevent or limit fastener **144** rotation within the tube **145,** as depicted in a cross-sectional view in Figure 51. With the guidance of the orientation line **153** or the handle **123,** the surgeon can avoid deploying the anchors **113** toward the direction of sensitive areas. Furthermore, to indicate the depth of device insertion, penetration markers **116** can be drawn on the fastener deployment tube **145,** as shown in Figure 50, and also on the disc compressor **118,** as an important verifiable reference point to the surgeon.

It is possible to add a joint or link in the rod **114** to provide extra flexibility and rotation between two anchoring ends of the tissue fastener **144.** Figure 52 depicts a mid-longitudinal view of a ball joint **152** between two sections of fastener rods **114** to allow rotation and flexibility. The ball **150** extends from one section of the rod **114** and is housed in a ball coupling **151** screwed onto or formed in another section of the rod **114.**

Since the guiding techniques for inserting the tissue fastener **144** into the bulging or herniated discs **100** are similar to the ones used in relatively low risk percutaneous nuclectomy procedures and routine diagnostic discography for determining herniations, the methods used in fastening bulging or herniated discs **100** should also be low in risk and complications. However, the success of percutaneous nuclectomies depends on the condition of the nucleus pulposus channels leading to the bulge. If the channels are closed, which is often the case, evacuation of the nucleus pulposus within the center of the vertebral disc does not affect the bulge and provides no benefit to the patient after the percutaneous nuclectomy procedure. Unlike the indirect and open channel dependent percutaneous nuclectomy procedure, the tissue fastener **144** is a direct mechanical device that hooks and locks the bulging annulus back into place. The results should be far more effective, predictable and longer lasting, with no higher risks or complications.

The tissue fastener **144** can be made with alloy, pure metal, polymer and/or composites. For super elastic and/or shape memory properties, nickel-titanium alloy can be used for the anchors **113** and/or the flexible rod **114.** A stainless steel tempered spring may also provide adequate resiliency, flexibility and elasticity, with sufficient biocompatibility for making the anchors **113** and/or the entire tissue fastener **144.** Many polymers, such as poly-sulfone, poly-ether-ether-ketone, DELRIN (acetal resin), polycarbonate, polyurethane, polypropylene, polyethylene and/or others, may have the physical and biological characteristics required to be a part of the tissue fastener **144.**

Although the counter tissue gripping anchors **113** on the fastener **144** can, and mostly likely will, resist device migration with time, after the tissue is adequately reattached or healed, it may be beneficial if a part or the entire tissue fastener **144** is biodegradable. Poly-lactate, poly-glycolate, collagen, elastin or other materials may provide the degradation profile to fasten then resorb after the tissue has reattached and healed.

To improve upon their performance, the tissue fastener **144** can be coated with radiopaque material, echogenic material, growth factor, antibiotic, analgesic, sealant, lubricant, nutrient and/or other substances.

The benefits of intervertebral disc **100** fastening include: (1) alleviating nerve **102** impingement, (2) minimizing spinal stenosis and/or (3) stabilizing intervertebral disc **100.** Annulus compression can be achieved with disc fastening devices, including the compression device **110,** disc restrictor **156,** tissue fastener **144** and any combinations of these devices.

### Disc Draining Device and Method to Relieve Low Back Pain

Percutaneous nuclectomy is often used to relieve low back pain by evacuating the nucleus pulposus **128** to decompress the intervertebral disc **100.** However, when the fluid builds up again in the future, low back pain returns.

Figure 53 depicts a disc drain tube **124** with nucleus pulposus inlets **125** and an outlet **126.** The drain tube **124** is inserted into a swollen intervertebral disc 100 through a drain delivery device **136** equipped with a plunger **117,** as shown in Figure 54. The construction of the drain delivery device **136** can be similar to the tissue fastener delivery device **120** in Figures 24-26. To aid with the insertion, penetration markers and/or curvature on the drain delivery device **136** can be helpful. The plunger **117** is held stationary while the drain delivery device **136** is withdrawn to deposit the disc drain tube **124** within the swollen disc **100.** Figure 55 depicts the uptake of the nucleus pulposus **128** drawn from the channels near the bulge of the disc **100** and the central reservoir through multiple inlets **125,** draining through the outlet **126** into the abdomen. As a result, the bulge **119** reduces in size, away from the previously impinged nerve **127,** as shown in Figure 55. The drain tube **124** can also be inserted anteriorly through the abdominal cavity to lower the risk of rupturing blood vessels, facilitating proper placement of the outlet **126** and creating only one incision.

Since the nucleus pulposus **128** is a viscoelastic gel with dynamic modulus ranging from 7 to 20 kPa, the pressure in the drained intervertebral disc **119** can be regulated or controlled by the diameter of the drain tube **124,** number of inlets **125,** sizes of the inlets **125** and/or the size of the outlet **126.** If the number of inlets **125** is few and diameters of the tube **124,** inlets **125** and outlet **126** are small, the disc pressure will remain high. On the other hand, if the inlets **125** are numerous and the diameters of the tube **124,** inlets **125** and outlets **126** are large, allowing significant nucleus pulposus **128** drainage to pass, the disc pressure will be low.

To improve disc pressure regulation, a connector **129** mounting a pressure sensing device **130** coupled with a nucleus pulposus **128** discharge gate **131** can be installed with the disc drain tube **124,** as depicted in Figure 56. As the pressure begins to build up within the disc **100,** the sensing device **130** triggers the gate **131** to open, allowing the nucleus pulposus **128** to escape out of the outlet **126** slits into the abdominal cavity. It is possible to build a disc drain tube **124** with a variety of pressure regulating designs to regulate and maintain the pressure within the intervertebral disc **119.**

The inlets **125** depicted in Figures 53 and 56 are deep and sunken into the disc drain tube **124** for three major functions: (1) to provide adequate drainage mainly from the central portion of the disc, (2) to minimize the diameter of the tube **124** and (3) to provide tissue ingrowth and/or gripping in the annular portion of the disc, thus preventing tube **124** migration with time. Tissue gripping elements can also be added onto the outer surface of the tube **124** to prevent migration.

The drain delivery device **136** and the drain tube **124** can also be made curved to avoid blood vessels, bone or nerves to promote proper drainage by tapping into channels of herniating nucleus pulposus **128,** using the drain tube **124** as a spigot to release the inflated bulge.

For various reasons, such as uncertain efficacy or degenerating discs, the disc drain tube **124** can be made biodegradable with poly-lactate, poly-glycolate, collagen, elastin or other degradable materials.

For long term pulposus **128** pressure regulation, metallic material, such as stainless steel, titanium or nickel-titanium, are suitable. Metallic material is preferred, especially if the distal end of the disc drain tube **124** is sharpened for disc puncturing capability. Numerous long lasting polymers, such as poly-ether-ether-ketone, polysulfone, polycarbonate, PTFE, polyurethane, DELRIN (acetal resin), polypropylene, polyethylene or others may meet adequate physical and biocompatible requirements.

To improve upon its performance, the disc drain tube **124** can be coated with a radiopaque compound, echogenic compound, growth factor, antibiotic, analgesic, sealant, lubricant, nutrient or other substances.

Although the single evacuation of nucleus pulposus **128** by the percutaneous nuclectomy procedure is well accepted due to the low risk in treating herniated discs **100,** the disc drain tube **124** provides a continually regulated and/or monitored drainage to prevent further swelling or herniation with a similar low risk level. The disc drain tube **124** may provide prolonged or even permanent relief for Types II to VI low back pain.

It is also possible to combine the benefits of the disc drain tube **124** with those of mechanical fastening using the compression device **110** for example, as indicated in Figure 57. The threaded rod **108** is made hollow with nucleus pulposus inlets **125** and an outlet **126,** and sealed near the disc restrainer **111** end. The mechanical fastening of the compression device **110** provides instant pain relief by alleviating the impinged nerve **102,** while the drainage reduces the swelling of the disc.

### Tendon Repair With Tissue Fastener

Tendon **138** rupture from bone commonly occurs in sports injuries and other accidents. Bone anchors (prior art) are most frequently used with tedious and delicate suture manipulative techniques to reattach torn tendon **138** back onto bone. To eliminate the trouble of suture manipulation, the tissue fastener **144** can be used to fasten the torn tendon **138** directly back onto bone.

To reattach tendon **138** with a tissue fastener **144,** a hole **140** is drilled or punctured into bone, then a tissue fastener deployment tube **145** with at least one tissue fastener **144** is inserted through the tendon **138** into the bone hole **140,** as indicated in Figure 58. A tissue-manipulating device **146** equipped with tissue manipulating elements **147** is used to position the tendon **138** onto the bone, as shown in Figure 59. To deploy the tissue fastener **144,** the tissue fastener deployment tube **145** is withdrawn while the plunger **117** and the tissue-manipulating device **146** are held stationary. As a result, the distal half of the tissue fastener **144** anchors in the bone hole **140** and the proximal half anchors in the tendon **138,** as depicted in Figure 60. Within weeks, the tendon **138** can, and most likely will, permanently reattach back onto the bone. Therefore, as mentioned previously, a biodegradable tissue fastener **144** can reattach tissue without the threat of device migration in the distant future or the creation of a stress riser, which weakens the bone.

For soft bone, such as the humerus **137** in the shoulder, as indicated in Figure 58, the tissue fastener deployment tube **145** can be sharpened as a needle, as shown in Figure 26, to puncture the humerus **137** without drilling. To ensure proper depth of insertion, penetration markers can be drawn on the outer surface of the tissue fastener deployment tube **145** to assist in the procedure. Similar delivery devices like the ones shown in Figures 24 and 26 can be used to puncture and manipulate tissues, then deliver and deploy the tissue fastener **144** with one hand, all through a tiny incision without the nuisance of suture manipulation.

### Meniscal Repair With Tissue Fastener

Currently, suture repair of meniscal tears frequently requires retraction of nerves and vessels, resulting in a long and painful recovery. Existing products, such as meniscal darts, screws and tacks, often creep and leave gaps in the supposed closure sites, not allowing the torn tissue to reattach and heal.

Both the tissue fastener **144** and the tissue fastener deployment needle **145** can be sized and configured to fasten and repair torn meniscus **135.** The tissue fastener deployment needle **145,** as shown in Figure 26, punctures the meniscal body with the guidance of an arthroscope, traversing the tear **139.** Penetration markers on the needle **145** can be helpful to determine the depth of needle **145** insertion. A tissue manipulating device **146** similar to the one used to position torn tendons **138,** as shown in Figure 59, can be utilized to approximate the torn tissue **139** back to the main body of the meniscus **135.** To deploy the tissue fastener **144,** the plunger **117** is held stationary while the fastener needle **145** is withdrawn from the meniscus **135.** When the restriction of the fastener deployment needle **145** has been lifted, the anchors **113** resiliently open into deployed positions, gripping, holding, grasping, trapping, hooking and/or fastening tissues at opposing ends of the tissue fastener **144,** as indicated in Figure 61. The anchors **113** from both ends of the fastener **144** have elastic gripping properties and are designed to resist pullout. As a result, the torn tissue **139** is elastically fastened and closed by the opposing grips of two sets of resilient anchors **113** of the tissue fastener **144,** as shown in Figure 61.

In meniscal repairs, the fastener deployment needle **145** is effective for both outside-in and inside-out approaches. The outside-in approach is to enter from the thick peripheral rim of the meniscus **135** toward the thin tapered portion of the meniscus **135.** The inside-out approach is to enter from the thin portion toward the thick rim. The inside-out approach is more frequently used by surgeons using sutures or meniscal tacks because with tears occurring more often in the posterior portion of meniscus **135;** it is less likely to disrupt vessels and nerves. The fasteners **144** and deployment needle **145** in this invention can accommodate both approaches.

Thin barbs on the meniscal tacks and darts, and the shallow thread of meniscal screws (prior art) can creep, leaving gaps under the enormous pressures exerted at the femorotibial joint. On the other hand, the long resilient and elastic anchors **113** of the tissue fastener provide continual spring-like closure forces rejoining the torn tissue **139,** thereby allowing the meniscus **135** to heal and serve its function. If the fastener **144** is made with biodegradable material, the fastener **144** will eventually degrade.

Although meniscal suture repair is believed to be reliable, it often requires large incisions, multiple entry points, retractors to pull aside blood vessels, nerves and/or expansion of joint space for passing and manipulating suture. Each of these retractions involves risks, post-surgical complications, prolonged healing time and increased pain and medical costs. Conversely, the tissue deployment needle **145** can deliver the spring-like tissue fastener **144** through a small incision for a quick recovery.

In brief summary, some of the possible benefits of the tissue fastener **144** and the delivery needle **145** are: (1) resilient tissue fastening, (2) minimal fastener migration, (3) minimally invasive, (4) accessible to deep body targets, (5) suture-free fastening, (6) attachable to bone, (7) minimal surgical space, (8) permanent or degradable fastening, (9) simple to use and (10) capable of manipulating tissue.

It is also to be understood that the present invention is by no means limited to the particular constructions disclosed herein and/or shown in the drawings, but also comprises any other modification, changes or equivalents within the scope of the claims. Many features have been listed with particular configurations, options, and embodiments. Any one or more of the features described may be added to or combined with any of the other embodiments or other standard devices to create alternate combinations and embodiments.

It should be clear to one skilled in the art that the current embodiments, materials, constructions, methods, tissues or surgical sites are not the only uses for which the invention may be used. Different materials, constructions, methods or designs for the compression device **110,** restricting device **156,** tissue fastener **144,** drain tube **124** or the delivery devices can be substituted and used. The use of this invention is also foreseen to compress, restrict, reattach or reconnect organ, muscle, skin, ligament, bone or other tissue. Nothing in the preceding description should be taken to limit the scope of the present invention. The full scope of the invention is to be determined by the appended claims.

## Claims

1. A compression device (110) for treating a bulging disc, herniated disc, intervertebral instability or spinal stenosis, said compression device comprising:
a rod (108) having a first end and a second end,
a toggle (107) pivotally connected with said first end of said rod, said toggle having a
delivery position and a deployed position,
**characterized in that** the compression device further comprises
a toggle deployment tube (109)
and a disc restraining member (111) attachable to said second end of said rod (108).

2. The compression device of claim 1 wherein, when said toggle is in said deployed position, said toggle is generally perpendicular to said rod, and when said toggle is in said delivery position, said toggle is generally parallel to said rod.

3. The compression device of claim 1 wherein said toggle comprises:
a first generally semi-cylindrical portion,
and a second generally semi-cylindrical portion,
wherein, in said deployed position, a first concave surface of said first generally semi-cylindrical portion faces generally towards a proximal end of said rod and a second concave surface of said second generally semi-cylindrical portion faces generally towards a distal end of said rod,
and wherein, in said delivery position, said first and second concave surfaces partially enclose said rod.

4. The compression device of claim 1 further comprising a nut attached to said first end of said rod and, preferably, wherein said nut is larger than an opening passing through said toggle.

5. The compression device of claim 1 wherein said toggle is connected with said first end of said rod by a pin.

6. The compression device of claim 5 wherein said toggle has a first member forming a first portion and a second member forming a second portion of said toggle and, preferably, wherein said first and second members move independently.

7. The compression device of claim 5 wherein said pin connects said toggle to said rod at a mid-portion of said toggle.

8. The compression device of claim 1 wherein said disc restraining member has a plurality of tissue gripping elements extending therefrom.

9. The compression device of claim 1 wherein said disc restraining member is resilient.

10. The compression device of claim 1 wherein said rod is threaded.

11. The compression device of claim 10 further comprising a nut holding said disc restraining member and, preferably, wherein said disc restraining member has a depression and said nut is at least partially recessed into said depression.

12. The compression device of claim 10 wherein said disc restraining member is located around a threaded tubular member sized and configured to mate with said rod and, preferably, wherein said tubular member has an internally threaded cavity and said cavity sized and configured to engage said rod.

13. The compression device of claim 10 wherein said rod has an internally threaded cavity extending therein and said disc restraining member is located around an externally threaded bolt, said bolt sized and configured to engage said cavity.

14. The compression device of claim 1 wherein at least a portion of said compression device is formed of a material chosen from the group of materials consisting of titanium, nickel-titanium, stainless steel, poly-ether-ether-ketone, acetal resin, polysulfone, polycarbonate, polypropylene, polyethylene, poly-lactate polymer and poly-glycolic polymer.

15. The compression device of claim 1 wherein said compression device is coated with a coating chosen from the group of coatings consisting of radiopaque material, echogenic material, growth factor, analgesic material, sealing material, blood clotting and antibiotic material.

16. The compression device of claim 1 wherein said compression device is flexible.

17. The compression device of claim 1 wherein said compression device is sized and configured to treat a dysfunctional intervertebral disc.

## Patentansprüche

1. Eine Druckvorrichtung (110) zur Behandlung einer gewölbten Scheibe, eines Bandscheibenvorfalls, einer intervertebraler Instabilität oder einer spinalen Stenosis, wobei die Druckvorrichtung umfaßt;
eine Stange (108) mit einem ersten Ende und einem zweiten Ende, einen Gelenkhebel (107), der drehbar mit dem ersten Ende der Stange verbunden ist, wobei der Gelenkhebel eine Zuführungsposition und eine eingesetzte Position aufweist,
**dadurch gekennzeichnet, daß** die Druckvorrichtung desweiteren umfaßt eine Gelenkhebeleinsatzröhre (109)
und ein Element (111) zum Zurückhalten einer Scheibe, das an dem zweiten Ende der Stange (108) befestigbar ist.

2. Druckvorrichtung nach Anspruch 1, wobei, wenn der Gelenkhebel in der eingesetzten Position ist, der Gelenkhebel hauptsächlich senkrecht zu der Stange ausgerichtet ist, und wenn der Gelenkhebel in der Zuführungsposition ist, ist der Gelenkhebel hauptsächlich parallel zu der Stange ausgerichtet.

3. Druckvorrichtung nach Anspruch 1, wobei der Gelenkhebel umfaßt:
einen ersten hauptsächlich halb-zylindrischen Teil,
und einen zweiten hauptsächlich halb-zylindrischen Teil,
wobei, in der eingesetzten Position, eine erste konkave Fläche des ersten hauptsächlich halb-zylindrischen Teils hauptsächlich in Richtung eines proximalen Endes der Stange gerichtet ist, und eine zweite konkave Fläche des zweiten hauptsächlich halb-zylindrischen Teils ist hauptsächlich in Richtung eines distalen Endes der Stange gerichtet,
und wobei, in der Zuführungsposition, die erste konkave Fläche und die zweite konkave Fläche teilweise die Stange umschließen.

4. Druckvorrichtung nach Anspruch 1, desweiteren umfassend eine Mutter, die an dem ersten Ende der Stange befestigt ist, und wobei vorzugsweise die Mutter größer als die Öffnung ist, die sich durch den Gelenkhebel erstreckt.

5. Druckvorrichtung nach Anspruch 1, wobei der Gelenkhebel mit dem ersten Ende der Stange durch einen Stift verbunden ist.

6. Druckvorrichtung nach Anspruch 5, wobei der Gelenkhebel ein erstes Element, das einen ersten Abschnitt bildet, und ein zweites Element aufweist, das einen zweiten Abschnitt des Gelenkhebels bildet, und wobei vorzugsweise das erste Element und das zweite Element sich unabhängig voneinander bewegen.

7. Druckvorrichtung nach Anspruch 5, wobei der Stift den Gelenkhebel mit der Stange an einem mittleren Abschnitt des Gelenkhebels verbindet.

8. Druckvorrichtung nach Anspruch 1, wobei das Element zum Zurückhalten einer Scheibe eine Vielzahl von sich hiervon erstreckenden Gewebehalteelementen aufweist.

9. Druckvorrichtung nach Anspruch 1, wobei das Element zum Zurückhalten einer Scheibe elastisch ist.

10. Druckvorrichtung nach Anspruch 1, wobei die Stange mit einem Gewinde versehen ist.

11. Vorrichtung nach Anspruch 10, desweiteren umfassend eine Mutter, die das Element zum Zurückhalten einer Scheibe hält, und wobei vorzugsweise das Element zum Zurückhalten einer Scheibe eine Vertiefung aufweist und die Mutter zumindest teilweise in die Vertiefung vertieft ist.

12. Druckvorrichtung nach Anspruch 10, wobei das Element zum Zurückhalten einer Scheibe um ein mit einem Gewinde versehenes rohrförmiges Element angeordnet ist, das ausgebildet und ausgelegt ist, um der Stange zu entsprechen, und wobei vorzugsweise das rohrförmige Element einen mit einem Innengewinde versehenen Hohlraum aufweist und der Hohlraum ausgelegt und ausgebildet ist, um mit der Stange in Eingriff zu treten.

13. Druckvorrichtung nach Anspruch 10, wobei die Stange einen mit einem Innengewinde versehenen Hohlraum aufweist, der sich hierin erstreckt, und wobei das Element zum Zurückhalten einer Scheibe um einen mit einem Außengewinde versehenen Bolzen angeordnet ist, wobei der Bolzen ausgelegt und ausgebildet ist, um mit dem Hohlraum in Eingriff zu treten.

14. Druckvorrichtung nach Anspruch 1, wobei mindestens ein Abschnitt der Druckvorrichtung aus einem Material gebildet ist, das aus der Gruppe von Materialien bestehend aus Titan, Nickeltitan, Edelstahl,
Polyetheretherketon, Acetalharz, Polysulfon, Polycarbonat, Polypropylen, Polyethylen, Polylactatpolymer und Polyglycolpolymer ausgewählt ist.

15. Druckvorrichtung nach Anspruch 1, wobei die Druckvorrichtung mit einer Schicht beschichtet ist, die aus der Gruppe von Beschichtungen bestehend aus röntgenstrahlundurchlässigem Material, echoerzeugendem Material, Zunahme, analgetischem Material, Dichtungsmaterial, Blutgerinnungsmaterial und antibiotischem Material ausgewählt ist.

16. Druckvorrichtung nach Anspruch 1, wobei die Druckvorrichtung flexibel ist.

17. Druckvorrichtung nach Anspruch 1, wobei die Druckvorrichtung ausgelegt und ausgebildet ist, um eine disfunktionelle Zwischenwirbelscheibe zu behandeln.

## Revendications

1. Dispositif de compression (110) pour traiter un disque bombé, un disque hernié, une instabilité intervertébrale ou une sténose du canal vertébral, ledit dispositif de compression comprenant :
- une tige (108) ayant une première extrémité et une seconde extrémité ;
- une bascule (107) connectée de façon pivotante à ladite première extrémité de ladite tige, ladite bascule ayant une position d'introduction et une position déployée,
**caractérisé par le fait que** le dispositif de compression comprend en outre
- un tube (109) de déploiement de bascule ; et
- un élément (111) de contention de disque pouvant être attaché à ladite seconde extrémité de ladite tige (108).

2. Dispositif de compression selon la revendication 1, dans lequel, lorsque ladite bascule est dans ladite position déployée, ladite bascule est généralement perpendiculaire à ladite tige, et lorsque ladite bascule est dans ladite position d'introduction, ladite bascule est généralement parallèle à ladite tige.

3. Dispositif de compression selon la revendication 1, dans lequel ladite bascule comprend :
- une première partie généralement semi-cylindrique ; et
- une seconde partie généralement semi-cylindrique,
dans lequel, dans ladite position déployée, une première surface concave de ladite première partie généralement semi-cylindrique est généralement tournée vers une extrémité proximale de ladite tige et une seconde surface concave de ladite seconde partie généralement semi-cylindrique est généralement tournée vers une extrémité distale de ladite tige,
et dans lequel, dans ladite position d'introduction, lesdites première et seconde surfaces concaves enferment partiellement ladite tige.

4. Dispositif de compression selon la revendication 1, comprenant en outre un écrou attaché à ladite première extrémité de ladite tige et, de préférence, dans lequel ledit écrou est plus grand qu'une ouverture passant à travers ladite bascule.

5. Dispositif de compression selon la revendication 1, dans lequel ladite bascule est connectée à ladite première extrémité de ladite tige par une broche.

6. Dispositif de compression selon la revendication 5, dans lequel ladite bascule a un premier élément formant une première partie et un second élément formant une seconde partie de ladite bascule et, de préférence, dans lequel lesdits premier et second éléments se déplacent indépendamment.

7. Dispositif de compression selon la revendication 5, dans lequel ladite broche connecte ladite bascule à ladite tige à une partie médiane de ladite bascule.

8. Dispositif de compression selon la revendication 1, dans lequel ledit élément de contention de disque a une pluralité d'éléments de prise de tissu s'étendant à partir de celui-ci.

9. Dispositif de compression selon la revendication 1, dans lequel ledit élément de contention de disque est élastique.

10. Dispositif de compression selon la revendication 1, dans lequel ladite tige est filetée.

11. Dispositif de compression selon la revendication 10, comprenant en outre un écrou maintenant ledit élément de contention de disque et, de préférence, dans lequel ledit élément de contention de disque a une dépression et ledit écrou est au moins partiellement encastré dans ladite dépression.

12. Dispositif de compression selon la revendication 10, dans lequel ledit élément de contention de disque est situé autour d'un élément tubulaire fileté dimensionné et configuré pour s'apparier avec ladite tige et, de préférence, dans lequel ledit élément tubulaire a une cavité taraudée et ladite cavité est dimensionnée et configurée pour engager ladite tige.

13. Dispositif de compression selon la revendication 10, dans lequel ladite tige a une cavité taraudée s'étendant dans celle-ci et ledit élément de contention de disque est situé autour d'un boulon fileté, ledit boulon étant dimensionné et configuré pour engager ladite cavité.

14. Dispositif de compression selon la revendication 1, dans lequel au moins une partie dudit dispositif de compression est formée d'une matière choisie dans le groupe des matières constituées par le titane, le nickel-titane, l'acier inoxydable, la poly-éther-éther-cétons, la résine d'acétal, la polysulfone, le polycarbonate, le polypropylène, le polyéthylène, le polylactate polymère et le polymère poly-glycolique.

15. Dispositif de compression selon la revendication 1, dans lequel ledit dispositif de compression est revêtu d'un revêtement choisi dans le groupe des revêtements constitués par une matière radio-opaque, une matière échogène, un facteur de croissance, une matière analgésique, une matière de scellement, une matière de coagulation du sang et une matière antibiotique.

16. Dispositif de compression selon la revendication 1, dans lequel ledit dispositif de compression est flexible.

17. Dispositif de compression selon la revendication 1, dans lequel ledit dispositif de compression est dimensionné et configuré pour traiter un disque intervertébral dysfonctionnel.
